(19) Europäisches Patentamt
European Patent Office
Office européen des brevets

(11) Publication number: **0 056 699**
**B1**

(12) **EUROPEAN PATENT SPECIFICATION**

(45) Date of publication of patent specification: **09.04.86**

(51) Int. Cl.[4]: **C 07 D 211/44**, C 08 K 5/34

(21) Application number: **82300137.5**

(22) Date of filing: **12.01.82**

(54) **Organic peroxides and use of same.**

(30) Priority: **16.01.81 IT 1916581**

(43) Date of publication of application:
**28.07.82 Bulletin 82/30**

(45) Publication of the grant of the patent:
**09.04.86 Bulletin 86/15**

(84) Designated Contracting States:
**BE CH DE FR GB LI NL**

(56) References cited:

**CHEMICAL ABSTRACTS, vol. 72, no. 8, February 23, 1970, page 8, abstract 32312f, COLUMBUS OHIO (US)**

**BULLETIN DE LA SOCIETE CHIMIQUE DE FRANCE, May-June 1975, C. FILLIATRE et al. "Application du système peroxyde de diacyle-hétérocycles N-substitué à la polymérisation radicalaire du chlorure de vinyle", pages 1152-1154**

(73) Proprietor: **AUSIMONT S.p.A.**
**31, Foro Buonaparte**
**Milan (IT)**

(72) Inventor: **Fontanelli, Renzo**
**19, Via Maiocchi**
**Milan (IT)**
Inventor: **Fontana, Alberto**
**47, Corso Lodi**
**Milan (IT)**
Inventor: **Cicchetti, Osvaldo**
**57, Via Sapri**
**Milan (IT)**
Inventor: **Sacrini, Egeo**
**Via Campiglio 9**
**Milan (IT)**

(74) Representative: **Whalley, Kevin et al**
**Marks & Clerk 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to organic peroxides and more particularly to organic peroxides having two peroxide functional groups and containing in the molecule an amino nitrogen atom, and to the use of the same in the cross-linking of plastomers, natural and synthetic elastomers and thermo-setting resins.

As is known, organic compounds of peroxide nature are particularly important as generators of free radicals and as a consequence as starters of radicalic polymerizations, as cross-linking agents of plastomers and as vulcanizing agents of natural and synthetic elastomers.

However, not all compounds of peroxide nature are employable in cross-linking as some types of peroxides are quite difficult to process due to their tendency to decompose, sometimes very strongly, at the operative temperature. Other compounds have a too short semi-life time, at the temperature of incorporation into the polymer, thus causing a certain degree of pre-cross-linking during the mixing step.

NL—A—137485 discloses 1,1-di(tert-butylperoxy)-3,3,5-trimethylcyclohexane, which may be employed commercially with an inert inorganic carrier or with a plasticizer for example.

It is an object of the present invention to provide a particular type of new peroxides containing in their molecule an amino nitrogen atom, having a good stability and a low volatility, and suitable as cross-linking agents of plastomers, natural and synthetic elastomers and thermosetting resins.

It has now been found that such object is achieved by employing peroxides having two peroxide functional groups and containing in the molecule an amino nitrogen atom.

The present invention provides an organic peroxide characterized by having two peroxide functional groups and containing in the molecule an amino nitrogen atom, and being of the general formula

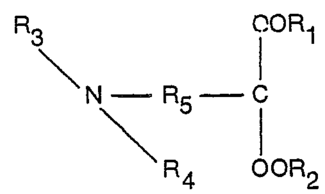

wherein:

$R_1$, $R_2$ are each t-butyl, t-amyl, t-octyl or cumyl;

$R_3$ is hydrogen, an alkyl radical having 1 to 8 carbon atoms, a cycloalkyl radical having 5—6 carbon atoms in the ring, an aryl radical or a benzyl radical; and

$R_4$ is trimethylene and $R_5$ is methylene or $R_4$ and $R_5$ are ethylene, wherein $R_4$ and $R_5$ are in both the possibilities each at least monosubstituted by methyl or phenyl.

It is apparent that, when $R_4$ = ethylene and $R_5$ = ethylene or when $R_4$ = trimethylene and $R_5$ = methylene, the resulting compound is an hexa-atomic ring (i.e. a derivative of piperidine).

Some examples of compounds according to the invention are:

(a) 4,4-di(t-butylperoxy)-2,2,6,6-tetramethylpiperidine
(b) 4,4-di(t-butylperoxy)-1,2,2,6,6-pentamethylpiperidine
(c) 4,4-di(t-butylperoxy)-2,6-diphenylpiperidine
(d) 4,4-di(t-amylperoxy)-2,2,6,6-tetramethylpiperidine
(e) 4,4-di(t-butylperoxy)-2,6-diphenyl-3,5-dimethylpiperidine
(f) 1-benzyl-4,4-di(t-butylperoxy)-2,6-di(methoxyphenyl)-3,5-dimethylpiperidine
(g) 4,4-di(cumylperoxy)-1,2,2,6,6-pentamethylpiperidine
(h) 4,4-di(cumylperoxy)-2,2,6,6-tetramethylpiperidine

The diperoxides according to the present invention exhibit a relatively high decomposition temperature and a good stability and a low volatility at higher temperatures than room temperature, which permits a good incorporation thereof into polymeric materials without causing any undesirable side-phenomena, such as pre-cross-linking and pre-vulcanization. It has been ascertained that the diperoxides of the present invention act as excellent cross-linking agents of plastomers, natural and synthetic elastomers and of thermosetting resins.

This class of nitrogenous peroxides exhibits the characteristics of being insensible to the action of minor amounts of acids, as the latter are neutralized by salification with the nitrogen atom, as a result of which they cannot decompose the peroxide part of the molecule.

There may be formed a composition comprising a plastomeric or a natural or synthetic elastomeric polymer and a peroxide compound according to the invention. A suitable plastomeric polymer is ethylene and a suitable elastomeric polymer is an ethylene-propylene copolymer.

Cross-linking is suitably effected at a temperature of from 100° to 200°C, preferably from 140° to 160°C, at a pressure of from 50 to 200 kg/cm$^2$, for from 5 to 60 minutes, preferably from 10 to 30 minutes.

The concentration by weight of the peroxide is preferably from 0.5 to 10%, more preferably from 2 to 5%, with respect to the plastomer.

Vulcanization is suitably effected at a temperature of from 140° to 190°C, preferably from 150° to 170°C, for from 5 to 200 minutes, preferably from 5 to 30 minutes, employing an ethylene-propylene copolymer.

The concentration by weight of the peroxide is preferably from 0.5 to 10%, more preferably from 2 to 5%, with respect to the elastomer.

Mixes having the following composition have proved to be particularly suitable:

| | |
|---|---|
| — ethylene-propylene copolymer | 100 parts by weight |
| — HAF carbon black (High Abrasion Furnace carbon black, according to ASTM No. 330). | 20—80 parts by weight |
| — ZnO | 1—10 parts by weight |
| — sulphur | 0.1—0.5 parts by weight |
| — peroxide | 0.005—0.02 moles. |

One particular preferred mix comprises the following components in the following amounts:

| | |
|---|---|
| — ethylene/propylene copolymer | 100 parts by weight |
| — carbon black | 50 parts by weight |
| — ZnO (or MgO) | 5 parts by weight |
| — sulphur | 0.3 parts by weight. |

There may be also formed a composition comprising a thermosetting resin and a peroxide compound according to the invention. The thermosetting resin may be suitably an unsaturated poly-ester resin.

Hardening is suitably effected at a temperature of from 50° to 170°C, preferably from 80° to 115°, especially when employing an unsaturated polyester resin.

The concentration by weight of the peroxide is preferably from 0.01 to 5% with respect to the resin.

The diperoxides according to the invention can be prepared by following known methods.

According to a preferred embodiment of the present invention, the diperoxides can be prepared by reacting a tertiary organic hydroperoxide with an amino organic compound containing a carbonyl group, at low temperature, in the presence of an inorganic or organic acid.

The invention will be further described with reference to the following illustrative Examples.

Example 1

Preparation of 4,4-di(t-butylperoxy)-2,2,6,6-tetramethylpiperidine.

15.5 g of triacetonamine (2,2,6,6-tetramethyl-4-piperidone) (0.1 mole) and 30 g of a 70% solution of t-butylhydroperoxide in water were introduced into a flask equipped with a stirrer and a thermometer. The temperature of the mixture was brought to about 15°C and over 2 hours there were introduced 88 g of 70% sulphuric acid, keeping the temperature at about 15—20°C. The mass was stirred for 2—3 hours at 15°C, whereupon 550 g of an aqueous solution of 10% sodium hydroxide were added in order to release the amine as a free base and to remove t-butylhydroperoxide, which was present in excess, as sodium salt; the peroxide so synthetized was then extracted with 100 ml of solvent (for example hexane). After separation of the phases, the organic phase was washed with water to remove unreacted triacetonamine, if any, and to neutralize the solution. After removal of the solvent, 25 g of a clear liquid that upon exposure to the air turns into a low-melting solid were obtained, which exhibited the following characteristics;

— melting point: 44°C

— density at 50°C: 0.9

— C: found = 64.05% (calculated = 64.32%), H: found = 11.30% (calculated = 11.11%), N: found = 4.30% (calculated = 4.41%)

— iodometric analysis: 99%

— gas-chromatographic analysis: 99%

— decomposition temperature: 130°C

— semi-life time at 122°C: 30 minutes (0.1 M in dodecane).

On the basis of N.M.R., I.R. and mass spectra and on the basis of centesimal analyses, the product was identified as 4,4-di(t-butylperoxy)-2,2,6,6-tetramethylpiperidine having the following formula;

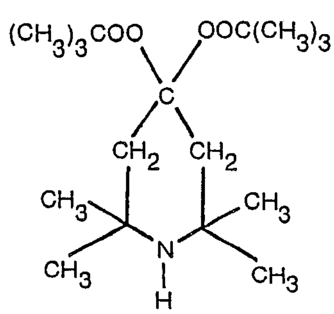

Example 2

Preparation of 4,4-di(t-butylperoxy)-1,2,2,6,6-pentamethylpiperidine.

The synthesis was conducted by operating according to the procedure described in Example 1, using, as a starting ketone, N-methyl-triacetonamine (1,2,2,6,6-pentamethyl-4-piperidone) (16.9 g = 0.1 mole).

There were obtained 15 g of a light yellow liquid which exhibited the following characteristics;

— C: found = 64.74% (calculated = 65.22%), H: found = 11.06% (calculated = 11.24%), N: found = 4.06% (calculated = 4.22%)
— iodometric analysis: 98%
— gas-chromatographic analysis: 98%
— decomposition temperature: 130°C
— semi-life time at 121°C: 30 minutes (0.1 M in dodecane).

On the basis of N.M.R., I.R. and mass spectra and on the basis of centesimal analyses, the product was identified as 4,4-di(t-butylperoxy)-1,2,2,6,6-pentamethylpiperidine having the following formula:

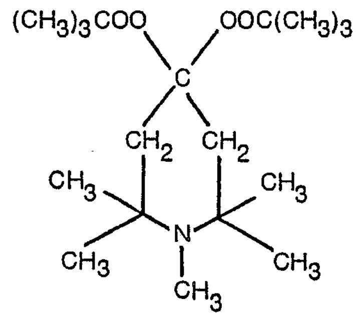

Example 3

Preparation of 4,4-di(t-butylperoxy)-2,6-diphenylpiperidine.

The synthesis was conducted by operating according to the procedure described in Example 1, using, as a starting ketone, 2,6-diphenyl-4-piperidone (25 g = 0.1 mole). The product obtained (5 g) was crystallized from methanol and exhibited the following characteristics:

— white crystalline solid
— melting point: 118°C
— C: found = 72.51% (calculated = 72.61%), H: found = 8.68% (calculated = 8.53%), N: found = 3.12% (calculated = 3.38%)
— iodometric analysis: 99%
— decomposition temperature: 130°C
— semi-life at 123°C: 30 minutes (0.1 M in dodecane).

On the basis of N.M.R., I.R. spectra and on the basis of centesimal analyses, the product was identified as 4,4-di(t-butylperoxy)-2,6-diphenylpiperidine having the following formula:

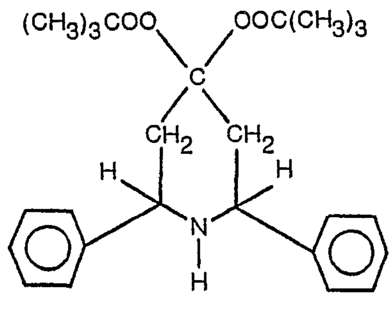

Example 4

Preparation of 4,4-di(t-amylperoxy)-2,2,6,6-tetramethylpiperidine.

15.5 g of triacetonamine (2,2,6,6-tetramethyl-4-piperidone) (0.1 mole) and 62 g of a 34% solution of t-amylhydroxyperoxide in hexane were introduced into a flask equipped with a stirrer and a thermometer. The temperature of the mixture was brought to about 15°C and over 90 minutes there were introduced 60 g of 70% sulphuric acid, keeping the temperature at about 15—20°C. The mass was stirred for about 6 hours at 15°C, whereupon 400 g of an aqueous solution of 10% sodium hydroxide were added in order to release the amine as a free base.

After separation of the phases, the organic phase was washed with water to remove unreacted triacetonamine, if any, and to neutralize the solution.

After removal of the solvent 10 g of a light yellow liquid were obtained, which exhibited the following characteristics:

— C: found = 65.08% (calculated = 66.04%), H: found = 11.10% (calculated = 11.37%), N: found = 4.30% (calculated = 4.05%)
— iodometric analysis: 95%
— gas-chromatographic analysis: 93%
— decomposition temperature: 100°C
— semi-life time at 121°C: 30 minutes (0.1 M in dodecane).

On the basis of N.M.R., I.R. and mass spectra and on the basis of centesimal analyses, the product was identified as 4,4-di(t-amylperoxy)-2,2,6,6-tetramethylpiperidine having the following formula:

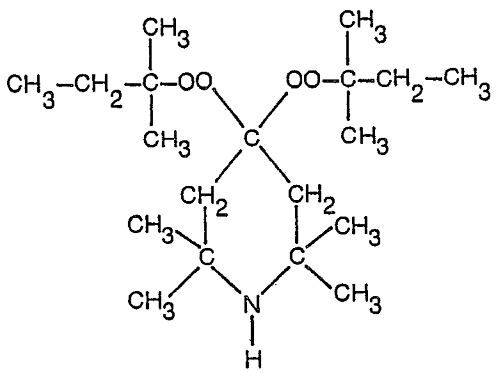

Example 5

Cross-linking

Cross-linking tests were carried out on polyethylene-based mixes of the type Fertene UK5-1830 (registered Trade Mark of Montedison S.p.A.) having a density = 0.917, and a grade = 1.2 g/10 min. at 190°C (ASTM D 1238), additioned with 0.01 moles of peroxide for 100 g of polyethylene.

Table 1 shows the conditions under which the cross-linking was effected as well as the cross-linking degree so obtained by using peroxides prepared according to Examples 1 and 2 in comparison with Trigonox 29/40 (registered Trade Mark of AKZO Co.) consisting of 40% of 1,1-di(t-butylperoxy)-3,3,5-trimethylcyclohexane and 60% of an inert organic carrier (see NL—A—137485).

The polyethylene cross-linking degree was measured by determining the solubility of the polymer in an organic solvent (decaline), according to the procedure prescribed by method ASTM D 2765. The polylethylene not cross-linked was thoroughly soluble in the solvent.

TABLE 1
CROSS-LINKING

| Composition | | TRIGONOX 29/40 1) | A* | B** | in the absence of peroxide |
|---|---|---|---|---|---|
| low density polyethylene | parts by weight | 100 | 100 | 100 | 100 |
| peroxide moles | | 0.01 | 0.01 | 0.01 | — |
| Cross-linking in press | | 30 minutes at 150°C | | | |
| undissolved polyethylene | g/100 g | 63.5 | 66.2 | 67.5 | thoroughly soluble |
| yield strength | Kg/cm² | 53.4 | 52.3 | 50.8 | 57.9 |
| tensile strength | Kg/cm² | 148 | 158 | 165 | 73 |
| elongation at break | % | 252 | 258 | 265 | 150 |

Notes:
A* 4,4-di(t-butylperoxy)-2,2,6,6-tetramethylpiperidine.
B** 4,4-di(t-butylperoxy)-1,2,2,6,6-pentamethylpiperidine.
1) Registered Trade mark.

Example 6

Vulcanization

Vulcanization tests were effected on mixed based on ethylene-propylene copolymer DUTRAL CO 054 (registered Trade Mark of Montedison S.p.A.) consisting of 55% by moles of ethylene — Mooney ML (1—14) viscosity at 100°C = 45.

The conditions under which vulcanization was carried out as well as the rheological and physical characteristics of the vulcanized products obtained by using the peroxides prepared in Examples 1, 2, 3 and 4 for vulcanization times of 20 minutes at a temperature of 150°C, in comparison with Trigonox 29/40, are given in Table 2.

TABLE 2

VULCANIZATION

| COMPOSITION | | TRIGONOX 29/40[1] | A(*) | B(*) | C(*) | D(*) |
|---|---|---|---|---|---|---|
| Dutral CO 054 | parts by weight | 100 | 100 | 100 | 100 | 100 |
| Carbon black HAF[1] | " " " | 50 | 50 | 50 | 50 | 50 |
| ZnO | " " " | 5 | 5 | 5 | 5 | 5 |
| S | " " " | 0.3 | 0.3 | 0.3 | 0.3 | 0.3 |
| Peroxide moles | | 0.01 | 0.01 | 0.01 | 0.01 | 0.01 |
| Vulcanization in press | | 20 minutes at 150°C | | | | |
| *Starting characteristics* | | | | | | |
| tensile strength | Kg/cm² | 203 | 204 | 215 | 196 | 160 |
| Elongation at break | % | 306 | 293 | 300 | 313 | 450 |
| Modulus 200% | Kg/cm² | 95 | 106 | 97 | 96 | 65 |
| Tearing resistance | Kg/cm | 36 | 37 | 36 | 37 | 45 |
| Shore A hardness | | 71 | 70 | 71 | 69 | 65 |
| *Characteristics after hardening in oven (7 days at 125°C)* | | | | | | |
| △ tensile strength | % | −3.9 | −3.6 | −3.7 | −1.1 | −6.2 |
| △ elongation at break | % | +12.6 | +12 | +11.2 | +8.3 | +18 |
| △ modulus 200 | % | −14.4 | −12 | −11.5 | −16.6 | −20 |
| Shore A hardness (variation in points) | | 0 | +1 | −1 | 0 | n.d. |
| Compression set % (70 hours at 100°C) | | 20 | 15 | 20 | 15 | 40 |
| Mooney scorch ($t_{10}$) minutes | | 6′ 45″ | 6′ 45″ | 7′ | 6′ 30″ | 8′ |

1) High Abrasion Furnace carbon black according to ASTM No. 330.
2) Registered Trade mark.

Notes to Table 2
A(*): 4,4-di(t.butylperoxy)-2,2,6,6-tetramethylpiperidine
B(*): 4,4-di(t.butylperoxy)-1,2,2,6,6-pentamethylpiperidine
C(*): 4,4-di(t.butylperoxy)-2,6-diphenylpiperidine
D(*): 4,4-di(t.amylperoxy)-2,2,6,6-tetramethylpiperidine.

Example 7

Hardening of unsaturated polyester resins.

Hardening tests of unsaturated polyester resins were carried out on a resin having the following composition:

| | |
|---|---|
| phthalic anhydride | 0.6 moles |
| maleic anhydride | 0.4 moles |
| propylene glycol | 1.05 moles ppm |
| hydroquinone | 150 ppm |
| styrene | approximately 36% calculated on the basis of the finished product. |

Such a resin, after addition with 1% of peroxide, was hardened at temperatures ranging from 80° to 115°C; both the maximum temperature attained by the resin during the hardening step and the time required to reach such temperature (hardening time) were taken.

The results were given in Table 3, where the peroxide 4,4-di(t-butylperoxy)-2,2,6,6-tetramethylpiperidine is compared with Trigonox 29 B/50 (commercial peroxide consisting of 50% of 1,1-di(t-butylperoxy)-3,3,5-trimethylcyclohexane and 50% of plasticizer).

TABLE 3

HARDENING OF THE UNSATURATED POLYESTER RESINS

| bath temperature °C | max. exothermic temperature | | hardening time | |
|---|---|---|---|---|
| | TRIGONOX[1] 29 B/50 | A (*) | TRIGONOX[1] 29 B/50 | A (*) |
| 80 | 190 | 95 | 22 | 95 |
| 90 | 205 | 207 | 15 | 40 |
| 100 | 207 | 212 | 8 | 21 |
| 115 | 210 | 212 | 4 | 9 |

Note:
A (*) : 4,4-di(t.butylperoxy)-2,2,6,6-tetramethylpiperidine.
1) Registered Trade mark.

**Claims**

1. An organic peroxide characterized by having two peroxide functional groups and containing in the molecule an amino nitrogen atom, and being of the general formula

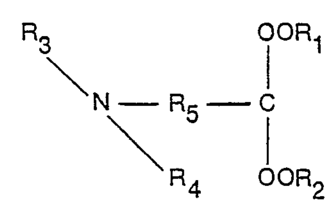

wherein:

$R_1$, $R_2$ are each t-butyl, t-amyl, t-octyl or cumyl;

$R_3$ is hydrogen, an alkyl radical having 1 to 8 carbon atoms, a cycloalkyl radical having 5—6 carbon atoms in the ring, an aryl radical or a benzyl radical; and

$R_4$ is trimethylene and $R_5$ is methylene or $R_4$ and $R_5$ are ethylene, wherein $R_4$ and $R_5$ are in both the possibilities each at least monosubstituted by methyl or phenyl.

2. 4,4-di(t-butylperoxy)-2,2,6,6-tetramethylpiperidine.
3. 4,4-di(t-butylperoxy)-1,2,2,6,6-pentamethylpiperidine.
4. 4,4-di(t-butylperoxy)-2,6-diphenylpiperidine.
5. 4,4-di(t-amylperoxy)-2,2,6,6-tetramethylpiperidine.
6. 4,4-di(t-butylperoxy)-2,6-diphenyl-3,5-dimethylpiperidine.

7. 1-benzyl-4,4-di(t-butylperoxy)-2,6-di(methoxyphenyl)-3,5-dimethylpiperidine.

8. 4,4-di(cumylperoxy)-1,2,2,6,6-pentamethylpiperidine.

9. 4,4-di(cumylperoxy)-2,2,6,6-tetramethylpiperidine.

10. A composition characterized by comprising a plastomeric or a natural or synthetic elastomeric polymer and a compound as claimed in any of Claims 1 to 9.

11. A composition as claimed in Claim 10, characterized in that the polymer is polyethylene or an ethylene-propylene copolymer.

12. A composition as claimed in Claim 10 or 11, characterized in that the amount by weight of peroxide cross-linking agent or peroxide vulcanizing agent employed is from 0.5 to 10% with respect to the plastomer or elastomer respectively.

13. A composition as claimed in Claim 12, characterized in that the amount by weight of peroxide cross-linking agent or peroxide vulcanizing agent employed is from 2 to 5% with respect to the plastomer or elastomer respectively.

14. A composition as claimed in Claim 11, characterized by comprising the following components in the following amounts:

| | |
|---|---|
| ethylene/propylene copolymer | 100 parts by weight |
| carbon black | 50 parts by weight |
| ZnO or MgO | 5 parts by weight |
| sulphur | 0.3 parts by weight |

15. A composition characterized by comprising a thermosetting resin and a compound as claimed in any of Claims 1 to 9.

16. A composition as claimed in Claim 15, characterised in that the thermosetting resin is an unsaturated polyester resin.

17. A composition as claimed in Claim 15 or 16, characterized in that the amount by weight of peroxide employed is from 0.01 to 5% with respect to the resin.

18. A process for cross-linking the composition according to Claim 10, characterized in that the cross-linking is effected at a temperature of from 100° to 200°C, at a pressure of from 50 to 200 $kg/cm^2$, for from 5 to 60 minutes.

19. A process as claimed in Claim 18, characterized in that the cross-linking is effected at a temperature of 140 to 160°C.

20. A process as claimed in Claim 18 or 19, characterized in that the cross-linking is effected for 10 to 30 minutes.

21. A process for vulcanizing the composition according to Claim 11, characterized in that the vulcanizing is effected at a temperature of from 140° to 190°C, for from 5 to 200 minutes.

22. A process as claimed in Claim 21, characterized in that the vulcanizing is effected at a temperature of from 150 to 170°C.

23. A process as claimed in Claim 21 or 22, characterized in that the vulcanizing is effected for from 5 to 30 minutes.

24. A process for hardening the composition according to Claim 15 or 16, characterized in that the hardening is effected at a temperature of from 50° to 170°C.

25. A process as claimed in Claim 24, characterized in that the hardening is effected at a temperature of from 80° to 115°C.

26. The use of a diperoxide according to any of Claims 1 to 9, as a cross-linking agent for a plastomer, natural or synthetic elastomer or unsaturated polyester resin.

**Patentansprüche**

1. Organisches Peroxid, dadurch gekennzeichnet, daß es zwei funktionelle Peroxidgruppen aufweist und im Molekül ein Aminostickstoffatom enthält und die allgemeine Formel hat

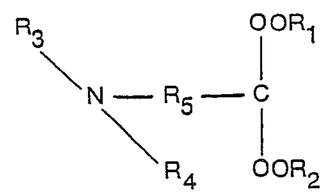

worin

$R_1$, $R_2$ jeweils t-Butyl, t-Amyl, t-Octyl oder Cumyl sind;

$R_3$ Wasserstoff, ein Alkylrest mit 1 bis 8 Kohlenstoffatomen ein Cycloalkylrest mit 5 bis 6 Kohlenstoffatomen im Ring, ein Arylrest oder ein Benzylrest ist; und

$R_4$ Trimethylen und $R_5$ Methylen ist oder $R_4$ und $R_5$ sind Ethylen, worin $R_4$ und $R_5$ in beiden Fällen jeweils mindestens durch Methyl oder Phenyl monosubstituiert sind.

2. 4,4-Di-(t-butylperoxy)-2,2,6,6-tetramethylpiperidin.
3. 4,4-Di-(t-butylperoxy)-1,2,2,6,6-pentamethylpiperidin.
4. 4,4-Di-(t-butylperoxy)-2,6-diphenylpiperidin.
5. 4,4-Di-(t-amylperoxy)-2,2,6,6-tetramethylpiperidin.
6. 4,4-Di-(t-butylperoxy)-2,6-diphenyl-3,5-dimethylpiperidin.
7. 1-Benzyl-4,4-di-(t-butylperoxy)-2,6-di-(methoxyphenyl)-3,5-dimethylpiperidin.
8. 4,4-Di-(cumylperoxy)-1,2,2,6,6,-pentamethylpiperidin.
9. 4,4-Di-(cumylperoxy)-2,2,6,6-tetramethylpiperidin.

10. Zusammensetzung, dadurch gekennzeichnet, daß sie ein plastomeres oder ein natürliches oder synthetisches elastomeres Polymer und eine Verbindung gemäß einem der Ansprüche 1 bis 9 umfaßt.

11. Zusammensetzung nach Anspruch 10, dadurch gekennzeichnet, daß das Polymer Polyethylen oder ein Ethylen-Propylen-Copolymer ist.

12. Zusammensetzung nach Anspruch 10 oder 11, dadurch gekennzeichnet, daß die Gewichtsmenge des verwendeten Peroxidvernetzungsmittels oder Peroxidvulkanisierungsmittels von 0,5 bis 10%, bezogen auf das Plastomer bzw. Elastomer, beträgt.

13. Zusammensetzung nach Anspruch 12, dadurch gekennzeichnet, daß die Gewichtsmenge des verwendeten Peroxidvernetzungsmittels oder Peroxidvulkanisierungsmittels von 2 bis 5%, bezogen auf das Plastomer bzw. Elastomer, beträgt.

14. Zusammensetzung nach Anspruch 11, dadurch gekennzeichnet, daß es die folgenden Komponenten in den folgenden Mengen umfaßt:

| | |
|---|---|
| Ethylen/Propylen-Copolymer | 100 Gew.-Teile |
| Ruß | 50 Gew.-Teile |
| ZnO oder MgO | 5 Gew.-Teile |
| Schwefel | 0,3 Gew.-Teile |

15. Zusammensetzung, dadurch gekennzeichnet, daß sie ein wärmehärtendes Harz und eine Verbindung nach einem der Ansprüche 1 bis 9 umfaßt.

16. Zusammensetzung nach Anspruch 15, dadurch gekennzeichnet, daß das wärmehärtende Harz ein ungesättigtes Polyesterharz ist.

17. Zusammensetzung nach Anspruch 15 oder 16, dadurch gekennzeichnet, daß die Gewichtsmenge des verwendeten Peroxid von 0,01 bis 5% bezogen auf das Harz, beträgt.

18. Verfahren zum Vernetzen der Zusammensetzung nach Anspruch 10, dadurch gekennzeichnet, daß die Vernetzung bei einer Temperatur von 100 bis 200°C 5 bis 60 min, bei einem Druck von 50 bis 200 $kg/cm^2$ erfolgt.

19. Verfahren nach Anspruch 18, dadurch gekennzeichnet, daß die Vernetzung bei einer Temperatur von 140 bis 160°C erfolgt.

20. Verfahren nach Anspruch 18 oder 19, dadurch gekennzeichnet, daß die Vernetzung 10 bis 30 min lang erfolgt.

21. Verfahren zum Vulkanisieren der Zusammensetzung gemäß Anspruch 11, dadurch gekennzeichnet, daß die Vulkanisierung bei einer Temperatur von 140 bis 190°C 5 bis 200 min lang erfolgt.

22. Verfahren nach Anspruch 21, dadurch gekennzeichnet, daß die Vulkanisierung bei einer Temperatur von 150 bis 170°C erfolgt.

23. Verfahren nach Anspruch 21 oder 22, dadurch gekennzeichnet, daß die Vulkanisierung 5 bis 30 min lang erfolgt.

24. Verfahren zum Härten der Zusammensetzung gemäß Anspruch 15 oder 16, dadurch gekennzeichnet, daß die Härtung bei einer Temperatur von 50 bis 170°C erfolgt.

25. Verfahren nach Anspruch 24, dadurch gekennzeichnet, daß die Härtung bei einer Temperatur von 80 bis 115°C erfolgt.

26. Die Verwendung eines Diperoxides gemäß einem der Ansprüche 1 bis 9 als Vernetzungsmittel für ein Plastomer, ein natürliches oder synthetisches Elastomer oder ein ungesättigtes Polyesterharz.

**Revendications**

1. Un péroxyde organique caractérisé en ce qu'il comporte deux groupes fonctionnels péroxydiques et contient, dans la molécule, un atome d'azote d'un groupe amine et en ce qu'il présente la formule générale:

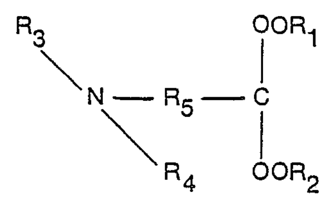

dans laquelle:

$R_1$, $R_2$ sont des radicaux t.butyle, t.amyle, t.octyle ou cumyle;

$R_3$ est un atome d'hydrogène, un radical alkyle comprenant 1 à 8 atomes de carbone, un radical cycloalkyle comprenant 5 ou 6 atomes de carbone dans le cycle, un radical aryle ou un radical benzyle; et

$R_4$ est le triméthylène et $R_5$ est le méthylène ou $R_4$ et $R_5$ sont de l'éthylène étant entendu que $R_4$ et $R_5$ ont, dans les deux cas, la possibilité d'être au moins monosubstitués par un groupe phényle ou méthyle.

2. 4,4-di(t.butylpéroxy)-2,2,6,6-tétraméthylpipéridine.
3. 4,4-di(t.butylpéroxy)-1,2,2,6,6-pentaméthylpipéridine.
4. 4,4-di(t.butylpéroxy)-2,6-diphénylpipéridine.
5. 4,4-di(t.amylpéroxy)-2,2,6,6-tétraméthylpipéridine.
6. 4,4-di(t.butylpéroxy)-2,6-diphényl-3,5-diméthylpipéridine.
7. 1-benzyl-4,4-di(t.butylpéroxy)-2,6-di(méthoxyphényl)-3,5-diméthylpipéridine.
8. 4,4-di(cumylpéroxy)-1,2,2,6,6-pentaméthylpipéridine.
9. 4,4-di(cumylpéroxy)-2,2,6,6-tétraméthylpipéridine.
10. Une composition caractérisée en ce qu'elle comprend un plastomère ou un polymère élastomère naturel ou synthétique et un composé ainsi que revendiqué dans l'une quelconque des revendications 1 à 9.
11. Une composition ainsi que revendiquée dans la revendication 10, caractérisée en ce que le polymère est le polyéthylène ou un copolymère éthylène-propylène.
12. Une composition ainsi que revendiquée dans la revendication 10 ou 11, caractérisée en ce que la quantité en poids d'agent de réticulation péroxydique ou d'agent de vulcanisation péroxydique employé est comprise entre 0,5 et 10% par rapport au plastomère ou à l'élastomère respectivement.
13. Une composition ainsi que revendiquée dans la revendication 12, caractérisée en ce que la quantité en poids d'agent de réticulation péroxydique ou d'agent de vulcanisation péroxydique employé est comprise entre 2 et 5% par rapport au plastomère ou à l'élastomère respectivement.
14. Une composition ainsi que revendiquée dans la revendication 11, caractérisée en ce qu'elle comprend les constituants suivants dans les quantités indiquées:

| | |
|---|---|
| copolymère éthylène-propylène | 100% en poids |
| noir de carbone | 50% en poids |
| ZnO ou MgO | 5% en poids |
| soufre | 0,3% en poids |

15. Une composition caractérisée en ce qu'elle comprend une résine thermodurcissable et un composé ainsi que revendiqué dans l'une quelconque des revendications 1 à 9.
16. Une composition ainsi que revendiquée dans la revendication 15, caractérisée en ce que la résine thermodurcissable est une résine polyester insaturée.
17. Une composition ainsi que revendiquée dans la revendication 15 ou 16, caractérisée en ce que la quantité ponderale de péroxyde employée est comprise entre 0,01 et 5% par rapport à la résine.
18. Un procédé de réticulation d'une composition selon la revendication 10, caractétrisé en ce que la réticulation est effectuée dans une température comprise entre 100°C et 200°C, à une pression comprise entre 50 et 200 kg/cm$^2$ pendant 5 à 60 minutes.
19. Un procédé ainsi que revendiqué dans la revendication 18, caractétrisé en ce que la réticulation est effectuée à une température comprise entre 140 et 160°C.
20. Un procédé ainsi que revendiqué dans les revendications 18 ou 19, caractérisé en ce que le réticulation est effectuée pendant 10 à 30 minutes.
21. Un procédé de vulcanisation d'une composition selon la revendication 11, caractérisé en ce que la vulcanisation est effectuée à une température comprise entre 140 et 190°C pendant 5 à 200 minutes.
22. Un procédé ainsi que revendiqué dans les revendication 21, caractérisé en ce que la vulcanisation est effectuée à une température comprise ente 150 et 170°C.
23. Un procédé ainsi que revendiqué dans les revendication 21 ou 22, caractérisé en ce que la vulcanisation est effectuée pendant 5 à 30 minutes.
24. Un procédé de durcissement d'une composition selon la revendication 15 ou 16, caractérisé en ce que le durcissement est effectué à une température comprise entre 50° et 170°C.
25. Un procédé ainsi que revendiqué dans la revendication 24, caractérisé en ce que le durcissement est effectué à une température comprise entre 80° et 115°C.
26. L'utilisation d'un dipéroxyde selon l'une quelconque des revendications 1 à 9, en tant qu'agent de réticulation pour un plastomère, un élastomère naturel ou synthétique ou une résine polyester insaturée.